# EUROPÄISCHE PATENTSCHRIFT

(11) **EP 1 220 838 B1**
(45) Veröffentlichungstag und Bekanntmachung des Hinweises auf die Patenterteilung: **03.11.2010**
(21) Anmeldenummer: 00982943.3
(22) Anmeldetag: 22.09.2000
(51) Int. Cl.: C07D 203/00

(54) **BIS(1-AZIRIDINO-1-HYDROXYIMINOMETHYL)-DERIVATE, VERFAHREN ZU DEREN HERSTELLUNG UND DEREN VERWENDUNG ALS ANTITUMORMITTEL**
1-AZIRIDINO-1-HYDROXYIMINOMETHYL-DERIVATIVES, METHOD FOR THE PRODUCTION THEREOF AND MEDICAMENTS CONTAINING SAID COMPOUNDS
DERIVES DE 1-AZIRIDINO-1-HYDROXYIMINOMETHYLE, PROCEDE DE FABRICATION, ET PRODUITS PHARMACEUTIQUES CONTENANT CES COMPOSES

(30) Priorität: 24.09.1999 DE 19947440
(43) Veröffentlichungstag der Anmeldung: 10.07.2002
(73) Patentinhaber: Kalvins, Prof. Dr., Ivars, 5052 Ikskile (LV)
(72) Erfinder: KALVINS, Ivars, 5052 Ikskile (LV); ADRIANOV, Viktor, LV-1063 Riga (LV); SHESTAKOVA, Irina, LV-1055 Riga (LV); KANEPE, Iveta, LV-1064 Riga (LV); DOMRACHEVA, Ilona, LV-1063 Riga (LV)
(74) Vertreter: Schubert, Klemens
(86) Internationale Anmeldenummer: PCT/DE2000/003441
(87) Internationale Veröffentlichungsnummer: WO 2001/021585

(56) Entgegenhaltungen:
- WO-A-84/04523
- US-A- 4 617 398
- US-A- 4 806 531
- US-A- 5 612 329
- EREMEEV A.V. ET AL.: "Synthesis and investigation of aziridino dioximes" CHEMISTRY OF HETEROCYCLIC COMPOUNDS (ENGL. TRANSLATION), US, PLENUM PRESS CO., NEW YORK, NY, Bd. 18, Nr. 4, 1982, Seiten 369-374, XP000986399 & KHIMIYA GETEROTSIKLICHESKIKH SOEDINENII , Nr. 4, 1982, Seiten 488-494, in der Anmeldung erwähnt

## Beschreibung

Die vorliegende Erfindung betrifft 1-Aziridino-1-hydroxyiminomethyl-Derivate, Verfahren zu deren Herstellung und diese Verbindungen enthaltende Arzneimittel.

Im Stand der Technik ist bisher nur ein Bisaziridinoxim der Formel 1 bekannt (Andrianov, V.G., Eremeev, A.V., Zh. Org. Khim. (1991), 27, 112-16; Eremeev, A.V., Piskunova, I.P., Andrianov, V.G., Liepins, E., Khim, Geterotsikl. Soedin (1982), (4) 488-94; Musluoglu, E., Ahsen, V., J. Chem. Research (S) (1999), 142-143).

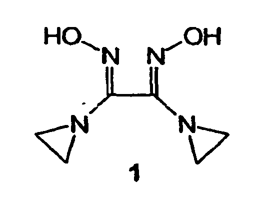

Von den biologischen Eigenschaften dieser Verbindung 1,1'-[1,2-bis-(Hydroximino-)-1,2-ethandiyl]bis-aziridin (1) oder deren Verwendung als Arzneimittel ist bisher nichts berichtet worden.

Ferner sind aus der DE-OS 21 32 598 u.a. Mono-Aziridinoxime bekannt, welche als Herbicide verwendet werden. In der WO 97/16439 werden gleichfalls Aziridinoxime beschrieben, welche zur Behandlung von Erkrankungen verwendet werden, die mit der Funktion des Chaperon-Systems in Verbindung stehen. Keinesfalls sind jedoch Bis-, Tris-oder gar Tetra-Aziridinoxime beschrieben worden.

In der WO 84/04523 werden Azidirino-Derivate von Tetrameren Cyclophosphazenen mit der allgemeine Formel N₄P₄Cl₈₋ₙAzₙ mit n= 1, 2, 3, 4, 5, 6, oder 7 beschrieben.

T. J. Bardos und M. E. Perlman beschreiben in der US 4,617,398 Phosphoaziridin-Verbindungen, die in der Behandlung von Tumoren hilfreich sind.

A. C. Sartorelli und T-.S. Lin beschreiben in der US 4,806,531 2,3-Bis-(Aziridinyl)-1,4-naphthoquinon-sulfonat-Derivat, die eine antineoplastische Aktivität aufweisen.

Weiterhin beschreibt P. S. Callery et al. in der US 5,612,329 Diaziridinylpolyamin als anti-Krebs Agens.

Aufgabe der vorliegenden Erfindung ist es, neue 1-Aziridino-1-hydroxyiminomethyl-Derivate der allgemeinen Formel I 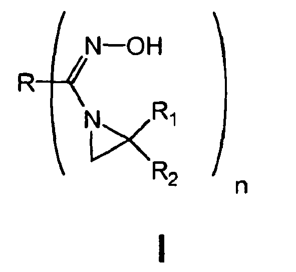 sowie ein Verfahren zu deren Herstellung zur Verfügung zu stellen. Eine weitere Aufgabe ist es, Arzneimittel die eine Verbindung der allgemeinen Formel I enthalten, zur Verfügung zu stellen.

In der allgemeinen Formel I bedeutet R einen beliebigen organischen Rest, der in der Lage ist, zwei Aziridinoxim-Gruppierungen, kovalent zu binden, und ausgewählt ist aus einer Einfachbindung, linearen oder verzweigten und gesättigten oder ungesättigten Alkanen oder Heteroalkanen mit bis zu 6 C-Atomen und mit bis zu vier Heteroatomen, C₃-C₈-Cycloalkanen, welche gegebenenfalls mit kurzkettigen C₁-C₆-Alkyl-, C₁-C₆-Alkoxy-, Nitro-, Amino-, monosubstituierten Amino-, und/oder Halogen-Resten substituiert sind, heterocyclischen Verbindungen mit 3 bis 6 Ringatomen und bis zu vier Heteroatomen, aromatischen Verbindungen mit bis zu 8 Ringatomen, welche gegebenenfalls mit Cyano-, Hydroxy-, kurzkettigen C₁-C₆-Alkyl-, C₁-C₆-Alkoxy-, Nitro-, Amino-, monosubstituierten Amino-, Trihalogenalkyl- und/oder Halogen-Resten substituiert sind und Heteroarylen mit 3 bis 7 Ringatomen und bis zu vier Heteroatomen, R₁ und R₂ stehen unabhängig voneinander für ein Wasserstoffatom oder eine -CH₃, -C₂H₅, -CN, -COOH, -COOCH₃, -CO0C₂H₅, -CONH₂ oder -C₆H₅ Gruppe stehen und n eine ganze Zahl 2 ist, mit der Ausnahme von 1,2-Bis-(Aziridin-N-yl)glyoxim und 1,2-Bis-(2-Methylaziridin-N-yl)glyoxim.

Besonders bevorzugt ist es dabei, daß der Grundkörper R ausgewählt ist aus Einfachbindung, Methyl, Ethan, Ethen, Ethin, Propan, Isopropan, Butan, Isobutan, sec-Butan, Pentan, Isopentan, Neopentan, Hexan, Azin, Cylopropan, Cyclobutan, Cyclopentan, Cyclohexan, Cycloheptan, Cyclooctan, Pyrrol, Pyrrolin, Pyrrolidin, Imidazol, Imidazolin, Pyrazolidin, Thiazol, Thiazolin, Thiazolidin, Isothiazol, Isothiazolin, Isothiazolidin, Benzothiazol, Furan, Dihyrofuran, Tetrahydrofuran, Benzofuran, Thiophen, Benzothiophen, Oxazol, Oxazolin, Oxazolidin, Benzoxazol, Isoxazol, Isoxazolin, Isoxazolidin, Piperidin, Piperazin, Pyrimidin, Morpholin, Dihydropyran, Tetrahydropyran, Pyridazin, Benzol, Furoxan, Imidazolidin, Pyrazol, Pyrazolin, Pyridin und dessen N-Oxid, Dihydropyridin, Pyrazin. Dabei ist klar, daß die jeweiligen Heteroatome an beliebigen Stellen im Ring positioniert sind.

Weiterhin bevorzugt ist es, daß R₁ und R₂ unabhängig von einander Wasserstoffatome sind oder einen Rest -CONH₂ darstellen.

Ganz besonders bevorzugt sind 2,6-Bis-(1-aziridino-1-hydroxyiminomethyl)pyridin (6), 1,4-Bis-(1-aziridino-1-hydroxyiminomethyl)benzol (7), 1,4-Di-(α-2-carbamoylaziridino-α-hydroxyiminomethyl)-benzol (8), 1,3-Bis-(1-aziridino-1-hydroxyiminomethyl)benzol (9), 1,3-Di-(α-2-carbamoylaziridino-α-hydroxyiminomethyl)-benzol (11), 2,6-Di-(α-2-carbamoylaziridino-α-hydroxyiminomethyl)-pyridin (12), 3,5-Bis-(1-aziridino-1-hydroxyiminomethyl)pyridin (13), 2,5-Bis-(1-aziridino-1-hydroxyiminomethyl)pyridin (14), 2,4-Bis-(1-aziridino-1-hydroxyiminomethyl)pyridin (15), 2,5,-Bis-(1-aziridino-1-hydroxyiminomethyl)furan (16), 3,4-Bis-[(aziridinyl-1)-hydroxyiminomethyl]furoxan (17), Bis-(2-methoxycarbonylaziridino)glyoxim (18), Bis-(2-carbamoylaziridino)glyoxim (19), 2,2'-Azinobis(1-aziridino-1-hydroxyimino)propan (20) und 2,2'-Azinobis[1-(2-carbamoylaziridino)-1-hydroxy-imino]propan (21).

Ein weiterer Gegenstand der vorliegenden Erfindung ist ein Verfahren zur Herstellung von erfindungsgemäßen 1-Aziridino-1-hydroxyiminomethyl-Derivaten, wobei man in an sich bekannter Weise eine Halogenverbindung der allgemeinen Formel II 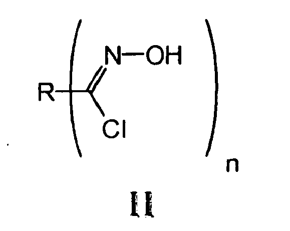 worin R und n die oben angegebene Bedeutung haben, mit einem Aziridin-Derivat der allgemeinen Formel III 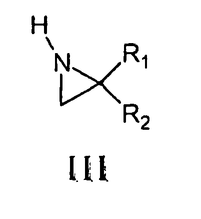 worin R₁ und R₂ die oben angegebene Bedeutung haben, umsetzt.

Die erfindungsgemäßen Verbindungen der Formel I lassen sich nach dem Formelschema 1 in an sich bekannter Weise herstellen. Zu diesem Zweck werden Nitrile der allgemeinen Formel IV durch Reaktion mit Hydroxylamin-Hydrochlorid in die Carboxamidoxime der allgemeinen Struktur VI überführt. Durch Diazotierung im salzsauren Milieu werden die chlorierten Oxime der Struktur II erhalten, die anschließend durch Reaktion mit Aziridinen der Formel III in die erfindungsgemäßen Verbindungen der Formel I überführt werden können. Alternativ kann, wie im Formelschema 1 angegeben, die Synthese ausgehend von den Carbonsäuren V über in der Literatur beschriebene Standardverfahren durchgeführt werden. Das experimentelle Verfahren ist für die Sequenz IV → VI → II → I in den Beispielen angegeben.

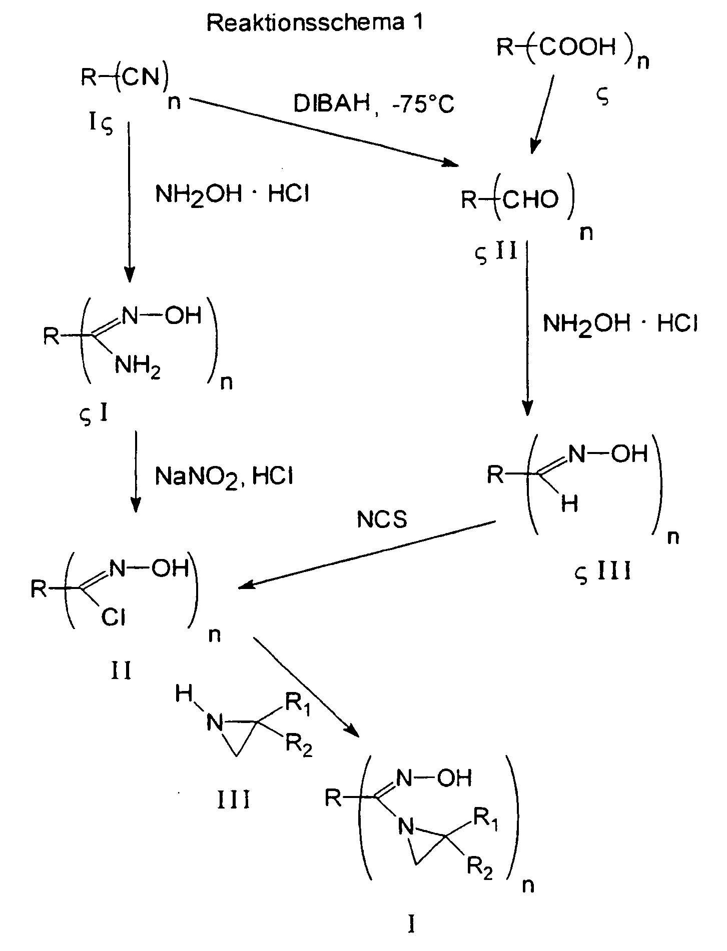

Ein weiterer Gegenstand der vorliegenden Erfindung sind Arzneimittel, gekennzeichnet durch einen Gehalt an einer Verbindung gemäß der allgemeinen Formel I.

Gegenstand der vorliegenden Erfindung sind auch Arzneimittel zur oralen, rektalen, subcutanen, intravenösen oder intramuskulären Applikation, die neben üblichen Träger- und Verdünnungsmitteln eine Verbindung der allgemeinen Formel I als Wirkstoff enthalten.

Geeignete Zubereitungsformen und deren Herstellung sind an sich bekannt und beispielsweise beschrieben in "Hagers Handbuch der pharmazeutischen Praxis", Springer Verlag - Berlin - Heidelberg, 1991, Band 2, S. 622ff.

Die Arzneimittel der Erfindung werden mit den üblichen festen oder flüssigen Trägerstoffen oder Verdünnungsmitteln und den üblicherweise verwendeten pharmazeutischtechnischen Hilfsstoffen entsprechend der gewünschten Applikationsart mit einer geeigneten Dosierung in bekannter Weise hergestellt. Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen oder Depotformen.

Selbstverständlich kommen auch parenterale Zubereitungen wie Injektionslösungen in Betracht. Weiterhin seien als Zubereitungen beispielsweise auch Suppositorien genannt.

Entsprechende Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelantine, Gleitmitteln wie Magnesiumstearat oder Talk und/oder Mitteln zur Erzielung eines Depoteffektes wie Carboxylpolymethylen, Carboxylmethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit dem erfindungsgemäßen Wirkstoff können zusätzlich geschmacksverbessernde Mittel wie Saccharin, Cyclamat oder Zucker sowie z. B. Aromastoffe wie Vanillin oder Orangenextrakt enthalten. Sie können außerdem Suspendierhilfsstoffe wie Natriumcarboxymethylcellulose oder Konservierungsstoffe wie p-Hydroxybenzoate enthalten. Wirkstoffe enthaltende Kapseln können beispielsweise hergestellt werden, indem man den Wirkstoff mit einem inerten Träger wie Milchzucker oder Sorbit mischt und in Gelatinekapseln einkapselt.

Geeignete Suppositorien lassen sich beispielsweise durch Vermischen mit dafür vorgesehenen Trägermitteln wie Neutralfetten oder Polyäthylenglykol bzw. deren Derivaten herstellen.

Selbstverständlich kommen auch transdermale therapeutische Systeme (TTS) in Betracht.

Die erfindungsgemäßen Verbindungen der allgemeinen Formel I zeigen antitumorale Wirksamkeit. In der Tabelle 1 sind die antitumoralen Aktivitäten einiger erfindungsgemäßer Verbindungen im Monolayer-Zytoxitätstest an ausgewählten Zellinien dargestellt. Überraschend ist dabei die geringe Empfindlichkeit von Fibroblasten und Endothelzellen bei der Anwendung der erfindungsgemäßen Verbindungen

Ein weiterer Gegenstand der vorliegenden Erfindung ist daher die Verwendung der 1-Aziridino-1-hydroxyiminomethyl-Derivate gemäß der allgemeinen Formel I zur Herstellung von Arzneimitteln zur Behandlung von Tumoren oder von Krebserkrankungen.

Ein weiterer Gegenstand der vorliegenden Erfindung ist die Verwendung von 1,1'-[1,2-bis-(Hydroximino-)-1,2-ethandiyl]bis-aziridin (1) zur Herstellung von Arzneimitteln zur Behandlung von Tumoren oder von Krebserkrankungen.

**Tabelle 1:**

| Antitumorale Wirksamkeit ausgewählter erfindungsgemäßer Verbindungen | | | | | | | | |
|---|---|---|---|---|---|---|---|---|
| Substanz | | 1 | 6 | 14 | 7 | 9 | 10 | 16 |
| IC₅₀ [µg/ml] | | | | | | | | |
| Organ/Zellinie | | | | | | | | |
| Kolon | HT29 | 0.486 | 0.117 | 0.200 | 0.258 | 0.329 | 0.670 | 0.481 |
| Magen | GXF 251L | 0.781 | 0.020 | 0.717 | 0.542 | 1.506 | 3.964 | 1.661 |
| Lunge | LXFL 529 | 0.441 | 0.027 | 0.006 | 0.038 | 0.063 | 0.100 | 0.099 |
| Brust | 401NL | 0.040 | 0.207 | 0.011 | 0.018 | 0.060 | 0.043 | 0.039 |
| Niere | 944LL | 0.923 | 0.115 | 0.198 | 0.348 | 0.788 | 0.750 | 1.359 |
| Uterus | 1138L | 0.173 | 0.014 | 0.034 | 0.038 | 0.066 | 0.111 | 0.073 |

Von der erfindungsgemäßen Verbindung 6 wurden an insgesamt 12 Zellinien (Tabelle 3) die mittleren IC₅₀-Werte im Vergleich zum Therapiestandard 5-Fluor-Uracil ermittelt (5FU) (siehe Tabelle 2).

Aus diesen Werten geht eine deutliche Überlegenheit der erfindungsgemäßen Verbindung gegenüber dem Therapiestandard hervor.

**Tabelle 2**

| Vergleich der antitumoralen Wirkung von (6) mit dem Theraoiestandard 5-Fluoruracil (5FU) | |
|---|---|
| **Verbindung** | **IC₅₀[µg/ml]** |
| (6) | 0,030 |
| 5FU | 0,054 |

**Tabelle 3**

| Verwendete Tumorzellinien | |
|---|---|
| **Tumor** | **Zellinie** |
| Brust | MAXF 401N |
| | MCF-7 |
| Kolon | HT29 |
| Magen | GXF251L |
| Lunge | LXFA 629L |
| | LXFE66L |
| | LXFL529 |
| Melanom | MEXF 462NL |
| | MEXF 514L |
| Eierstock | OVCAR3 |
| Niere | RXF 944L |
| Uterus | UXF 1138L |

Die nachfolgende Beispiele erläutern die Erfindung.

### Beispiele

### Beispiel 1

### Darstellung von 2,6-Bis-(1-aziridino-1-hydroxyimino-methyl)pyridin (6)

### Pyridin-2,6-dicarboxamidoxim

Zu einer Lösung von Hydroxylamin-Hydrochlorid (18,07 g; 26 mmol) und NaOH (10,40 g; 26 mmol) in H₂O (90 ml) wird unter starkem Rühren eine Lösung von Pyridin-2,6-dicarbonitril (12,9 g; 10mmol) in Ethanol (60 ml) getropft. Eine exotherme Reaktion tritt ein, anschließend wird 1,5 h bei 40-50°C weitergerührt. Nach Abkühlen wird der Niederschlag abfiltriert und mit H₂O gewaschen. Man erhält nach dem trockenen 16,5 g (85% d. Tr.) Produkt. M.p. 237-239°C. 1H-NMR (DMSO-d₆): δ 6,20 (4H, s, NH₂); 7,76 (3H, s, C₅H₃N); 9,76 (2H, s, OH), -CHN (%) gef.: C 43,6; H 4,5; N 35,9-ber: C 43,1; H 4,6; N 35,9.

Pyridin-2,6-dihydroxamic-dichlorid Zu einer gekühlten Lösung von Pyridin-2,6-dicarboxamidoxum (1,95 g; 10 mmol) in verdünnter HCl (20 ml conc. HCl + 8 ml H₂O) wird unter Rühren vorsichtig eine Lösung von NaNO₂ (1,78 g; 25 mmol) in H₂O (5 ml) getropft. Nach 1,5 h bei 0-10°C wird die Lösung 12h bei Raumtemperatur weiter gerührt. Anschließend wird der Niederschlag abfiltriert und mit H₂O gewaschen. Nach dem Trocknen erhält man 2,0 g (79% d. Tr.) Produkt. M.p. 168-170°C (Zers.),- ¹H-NMR (DMSO-D₆): δ 8,00 (3H, s, C₅H₃N); 12,7 (2H, s, OH).- CHN (%) gef. C 33,7; H 2,2; N 16,6 - ber.: C 33,3; H 2,2; N 16,7.

2,6-Bis-(1-aziridino-1-hydroxyiminomethyl)pyridin (6) Zu einer auf 0°C gekühlten Lösung von Aziridin (0,65 g; 15 mmol) und N(C₂H₅)₃ (2,0 g; 20mmol) in Acetonitril (20 ml) wird unter Rühren tropfenweise eine Suspension von Pyridin-2,6-dihydroxamicdichlorid (1,26 g; 5 mmol) in CH₃CN (20 ml) gegeben. Man rührt 90 min. nach und filtert vom ausgefallenen Triethylamin-Hydrochlorid ab. Das Filtrat wird im Vakuum eingeengt und mit Essigester versetzt. Man filtriert erneut und wäscht das Produkt mit CHCl₃ nach. Man erhält 0,76 g (60% d. Tr.) an Produkt. M.p. 194-196°C (Zers.). ¹H-NMR: δ 2,31 (8H, s, CH₂); 7,73 (3H, s, C₅H₃N); 10, 64 (2H, s, OH). CHN (%) gef.: C 52,4; H 5,3; N 27,5 (C₁₁H₁₃N₅O₂ x 0,25 H₂O) - ber. : C 52,5; H 5,4; N 27,8.

In analoger Weise erhält man die folgenden Verbindungen:

### Beispiel 2

### 1,4-Bis-(1-aziridino-1-hydroxyiminomethyl)benzol (7)

M.p. 220-222°C (Zers.). ¹H-NMR: δ 2,20 (8H, s, CH₂); 7,00 (4H, s, C₆H₄); 12,6 (2H, s, OH). CHN (%) gef.: C 58,3; H 5,9; N 22,4 (C₁₂H₁₄N₄O₂) - ber.: C 58,5; H 5,7; N 22,7.

### Beispiel 3

### 1,9-Di-(α-2-carbamoylaziridino-α-hydroxyiminomethyl)-benzol (8)

M.p. 248-250°C (Zers.). ¹H-NMR: δ 2,36 (4H, s, CH₂); 2, 82 (2H, m, CH); 7,16 und 7,47 (jeweils 2H, s, s, NH₂); 7,64 (4H, s, C₆H₄); 10,6 (2H, s OH). CHN (%) gef.: C 50,3; H 4,9; N 24,9 (C₁₄H₁₆N₆O₄) - ber.: C 50,6; H 4,8; N 25,3.

### Beispiel 4

### 1,3-Bis-(1-aziridino-1-hydroxyiminomethyl)benzol (9)

M.p. 179-181°C (Zers.). ¹H-NMR: δ 2,17 (8H, s, CH₂); 7, 31 (1H, t, C₆H); 7,62 (2H, d, C₆H₂); 8.11 (1H, s, C₆H); 11,3 (2H, s, OH). CHN (%) gef.: C 58,7; H 5,8; N 22,3 (C₁₂H₁₄N₄O₂) - ber.: C 58,5; H 5,7; N 22,7.

### Beispiel 1 5 (Referenzbeispriel)

### 1,3,5-Tris-(1-aziridino-1-hydroxyiminomethyl)benzol. (10)

M.p. >300°C (Zers.). ¹H-NMR: δ 2,16 (12H, s, CH₂); 8,00 (3H, s, C₆H₃); 11,4 (3H, s, OH). CHN (%) gef.: C 54,1; H 5,4; N 25,0 (C₁₅H₁₈N₆O₃) - ber.: C 54,5; H 5,5; N 25,4.

### Beispiel 6

### 1,3-Di-(α-2-carbamoylaziridino-α-hydroxyiminomethyl)-benzol (11)

M.p. 209-211°C (Zers.). ¹H-NMR: δ 2,38 (4H, m, CH₂); 3,02 (2H, m, CH); 7,16 und 7,42 (jeweils 2H, s, s, NH₂); 7,42 (1H, t, C₆H); 7,91 (1H, t, C₆H); 10,6 (2H, m, OH). CHN (%) gef.: C 45,9; H 5,3; N 22,8 (C₁₄H₁₆N₆O₄ x 2H₂O) - ber.: C 45,6; H 5,5; N 22,8.

### Beispiel 7

### 2,6-Di-(α-2-carbamoylaziridino-α-hydroxyiminomethyl)-pyridin (12)

M.p. 206-208°C (Zers.). ¹H-NMR: δ 2,38 (4H, m, CH₂); 2,96 (2H, m, CH); 7,11 und 7,40 (jeweils 2H, ss, NH₂); 7,76 (3H, s, C₅H₃N); 10,78 (2H, s, OH). CHN (%) gef.: C 46,6; H 4,6; N 29,0 (C₁₃H₁₅N₇O₄) - ber.: C 46,8; H 4,5; N 29,4.

### Beispiel 8

### 3,5-Bis-(1-aziridino-1-hydroxyiminomethyl)pyridin (13)

M.p. >300°C (Zers.). ¹H-NMR: δ 2,27 (8H, s, CH₂); 8,29 (1H, t, 4-C₅HN); 8,78 (2H, d, 2,6-C₅H₂N); 11,7 (2H, s, OH). CHN (%) gef.: C 53,7; H 5,1; N 28,2 (C₁₁H₁₃N₅O₂) - ber. : C 53,4; H 5, 3; N 28,3.

### Beispiel 9

### 2,5-Bis-(1-aziridino-1-hydroxyiminomethyl)pyridin (14)

M.p. 190-192°C (Zers.). ¹H-NMR: δ 2,22 (4H, s, CH₂); 2,26 (4H, s, CH₂); 7,76 (1H, d, C₅HN); 7,96 (1H, d, C₅HN); 8,78 (1H, s, C₅HN); 11,7 (2H, s, OH). CHN (%) gef.: C 53,8; H 5,2; N 28,0 (C₁₁H₁₃N₅O₂) - ber.: C 53,4; H 5,3; N 28,3.

### Beispiel 10

### 2,4-Bis-(1-aziridino-1-hydroxyiminomethyl)pyridin (15)

M.p. >300°C (Zers.). ¹H-NMR: δ 2,20 (8H, s, CH₂); 7,53 (1H, dd, C₅HN); 8,16 (1H, d, C₅HN); 8,51 (1H, d, C₅HN); 11,6 (1H, s, OH); 11,8 (1H, s, OH). CHN (%) gef.: C 53,4; H 5,5; N 28,0 (C₁₁H₁₃N₅O₂) - ber.: C 53,4; H 5,3; N 28,3.

### Beispiel 11

### 2,5,-Bis-(1-aziridino-1-hydroxyiminomethyl)furan (16)

M.p. 182-184°C (Zers.). ¹H-NMR: δ 2,22 (8H, s, CH₂); 6,78 (2H, s, C₄H₂0); 10,5 (2H, s, OH). CHN (%) gef.: C 47,3; H 5,6; N 22,1 (C₁₀H₁₂N₄O₄) - ber.: C 47,2; H 5,6; N 22,0.

### Beispiel 12

### 3,4-Bis-[(aziridinyl-1)-hydroxyiminomethyl]furoxan (17)

M.p. >300°C (Zers.). ¹H-NMR: δ 2,18 (4H, s, CH₂); 2,43 (4H, s, CH₂); 11,1 (1H, s, OH) ; 11,4 (1H, s, OH). CHN (%) gef.: C 38,2; H 4,2; N 32,9 (C₈H₁₀N₆O₄) - ber.: C 37,8; H 4,0; N 33,1.

### Beispiel 13

### Bis-(2-methoxycarbonylaziridino)glyoxim (18)

M.p. 212-214°C. ¹H-NMR: δ 2,36 (4H, m, CH₂); 2,96 (2H, m, CH); 3,62 (6H, s, CH₃); 10,71 (2H, s, OH). CHN (%) gef.: C 42,3; H 5,0; N 19,3 (C₁₀H₁₄N₄O₆) - ber.: C 42,0; H 4,9; N 19,6.

### Beispiel 14

### Bis-(2-carbamoylaziridino)glyoxim (19)

M.p. >300°C. ¹H-NMR: δ 2,28 (1H, m, CH); 2,40 (1H, m, CH); 2,83 (1H, m, CH); 7,09 und 7,24 (jeweils 1H, s, s, NH₂); 10,65 (1H, s, OH). CHN (%) gef.: C 37,1; H 4,8; N 32,1 (C₈H₁₂N₆O₄) - ber.: C 37,5; H 4,7; N 32,8.

### Beispiel 15

### 2,2'-Azinobis(1-aziridino-1-hydroxyimino)propan (20)

M.p. 172-174°C. ¹H-NMR: δ 1,91 (6H, s, CH₃); 2,20 (8H, s, CH₂); 10,9 (2H, s, OH). CHN (%) gef.: C 46,4; H 4,5; N 32,2 (C₁₀H₁₆N₆O₂ x 0,5 H₂O) - ber.: C 46,0; H 6,6; N 32,2.

### Beispiel 16

### 2,2'-Azinobis[1-(2-carbamoylaziridino)-1-hydroxyimino]-propan (21)

M.p. 242-244°C (Zers.). ¹H-NMR: δ 1,98 (6H, s, CH₃); 2,53 (2H, s, CH₂); 2, 53 (2H, m, CH₂); 2,89 (2H, m, CH); 7,04 und 7,22 (jeweils 2H, ss, NH₂); 11,02 (2H, s, OH). CHN (%) gef.: C 41,6; H 5,4; N 32,1 (C₁₂H₁₉N₈O₄ x 0,5 H₂O) - ber. : C 41,5; H 5, 5; N 32,3.

### Beispiel 19

Zur Untersuchung der antiproliferativen Eigenschaften der erfindungsgemäßen Verbindungen wurde ein modifizierter Propidium-Iodid-Assay (Dengler, W.A., Schulte, J, Berger, P.B., Mertelsmann, R., Fiebig, H.H.: Anti-Cancer Drugs 6, 522-532, (1995)) wie nachfolgend beschrieben durchgeführt:

Tumorzellen aus in der exponentiellen Wachstumsphase befindlichen Zellkulturen, (RPMI Medium, 10% FCS )wurden geerntet, gezählt und in 96 well Microtiterplatten (140µL Zell Suspension, 1x105 oder 5x104 Zellen/mL) überführt. Nach einer Zeitspanne von 24 h, in der die Zellen ihr exponentielles Wachstum wieder aufnahmen, wurden jeweils 10µL der in Medium gelösten Testsubstanzen zugefügt (Jede Testkonzentration wurde dreifach bestimmt).Nach 3-6 Tagen Inkubationszeit (in Abhängigkeit von der Verdoppelungsrate der Zellen) wurde das Kulturmedium gegen 200 µL eines frischen Mediums, welches Propidium- Iodid (25µg/mL) enthielt, ausgetauscht. Die Mikrotiterplatten wurden dann 24 Stunden bei -18°C gelagert, um einen kompletten Zelltod zu erreichen. Nach dem Auftauen der Platten wurde die Fluoreszenz mittels eines Millipore Cytoflour 2350 (Anregung 530 nm, Emission 620 nm) gemessen. Die IC₅₀-Werte der Testverbindungen wurden gemäß der publizierten Formel berechnet. Konnte eine IC₅₀ nicht innerhalb der untersuchten Dosis Einheiten bestimmt werden, wurde die jeweils niedrigste bzw. höchste untersuchte Konzentration für die Kalkulation benutzt.

## Patentansprüche

1. 1-Aziridino-1-hydroxyiminomethyl-Derivate der allgemeinen Formel I 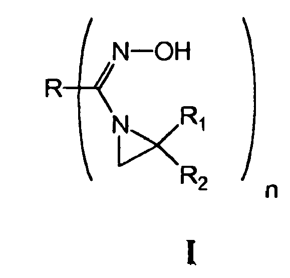 worin
R einen beliebigen organischen Rest bedeutet, der in der Lage ist, zwei Aziridinoxim-Gruppierungen kovalent zu binden, und ausgewählt ist aus
einer Einfachbindung, linearen oder verzweigten und gesättigten oder ungesättigten Alkanen oder Heteroalkanen mit bis zu 6 C-Atomen und mit bis zu vier Heteroatomen, C₃-C₈-Cycloalkanen, welche gegebenenfalls mit kurzkettigen C₁-C₆-Alkyl-, C₁-C₆-Alkoxy-, Nitro-, Amino-, monosubstituierten Amino-, und/oder Halogen-Resten substituiert sind,
heterocyclischen Verbindungen mit 3 bis 6 Ringatomen und bis zu vier Heteroatomen,
aromatischen Verbindungen mit bis zu 8 Ringatomen, welche gegebenenfalls mit Cyano-, Hydroxy-, kurzkettige C₁-C₆-Alkyl-, C₁-C₆-Alkoxy-, Nitro-, Amino-, monosubstituierten Amino-, Trihalogenalkyl- und/oder Halogen-Resten substituiert sind und
Heteroarylen mit 3 bis 7 Ringatomen und bis zu vier Heteroatomen,
R₁ und R₂ unabhängig voneinander für ein Wasserstoffatom oder eine -CH₃, -C₂H₅, -CN, -COOH, -COOCH₃, -COOC₂H₅, -CONH₂ oder -C₆H₅ Gruppe stehen,
n die ganze Zahl 2 ist,
mit der Ausnahme von 1,2-Bis-(Aziridin-N-yl)glyoxim und 1,2-Bis-(2-Methylaziridin-N-yl)glyoxim.

2. 1-Aziridino-1-hydroxyiminomethyl-Derivate gemäß Anspruch 1, **dadurch gekennzeichnet, daß** der Grundkörper R ausgewählt ist aus Einfachbindung, Methyl, Ethan, Ethen, Ethin, Propan, Isopropan, Butan, Isobutan, sec-Butan, Pentan, Isopentan, Neopentan, Hexan, Azin, Cylopropan, Cyclobutan, Cyclopentan, Cyclohexan, Cycloheptan, Cyclooctan, Pyrrol, Pyrrolin, Pyrrolidin, Imidazol, Imidazolin, Pyrazolidin, Thiazol, Thiazolin, Thiazolidin, Isothiazol, Isothiazolin, Isothiazolidin, Benzothiazol, Furan, Dihyrofuran, Tetrahydrofuran, Benzofuran, Thiophen, Benzothiophen, Oxazol, Oxazolin, Oxazolidin, Benzoxazol, Isoxazol, Isoxazolin, Isoxazolidin, Piperidin, Piperazin, Pyrimidin, Morpholin, Dihydropyran, Tetrahydropyran, Pyridazin, Benzol, Furoxan, Imidazolidin, Pyrazol, Pyrazolin, Pyridin und dessen N-Oxid, Dihydropyridin, Pyrazin.

3. 1-Aziridino-1-hydroxyiminomethyl-Derivate gemäß einem der voranstehenden Ansprüche, **dadurch gekennzeichnet, daß** R₁ und R₂ unabhängig von einander Wasserstoffatome sind oder einen Rest -CONH₂ darstellen.

4. 1-Aziridino-1-hydroxyiminomethyl-Derivate gemäß Anspruch 1, nämlich
2,6-Bis-(1-aziridino-1-hydroxyiminomethyl)pyridin (6),
1,4-Bis-(1-aziridino-1-hydroxyiminomethyl)benzol (7),
1,4-Di-(α-2-carbamoylaziridino-α-hydroxyiminomethyl)-benzol (8),
1,3-Bis-(1-aziridino-1-hydroxyiminomethyl)benzol (9)
1,3-Di-(α-2-carbamoylaziridino-α-hydroxyiminomethyl)-benzol (11),
2,6-Di-(α-2-carbamoylaziridino-α-hydroxyiminomethyl)-pyridin (12),
3,5-Bis-(1-aziridino-1-hydroxyiminomethyl)pyridin (13),
2,5-Bis-(1-aziridino-1-hydroxyiminomethyl)pyridin (14),
2,4-Bis-(1-aziridino-1-hydroxyiminomethyl)pyridin (15),
2,5,-Bis-(1-aziridino-1-hydroxyiminomethyl)furan (16),
3,4-Bis-[(aziridinyl-1)-hydroxyiminomethyl]furoxan (17),
Bis-(2-methoxycarbonylaziridino)glyoxim (18),
Bis-(2-carbamoylaziridino)glyoxim (19),
2,2'-Azinobis(1-aziridino-1-hydroxyimino)propan (20) und
2,2'-Azinobis[1-(2-carbamoylaziridino)-1-hydroxy-imino]propan (21)

5. Verfahren zur Herstellung von 1-Aziridino-1-hydroxyiminomethyl-Derivaten gemäß Anspruch 1, wobei man in an sich bekannter Weise eine Halogenverbindung der allgemeinen Formel II 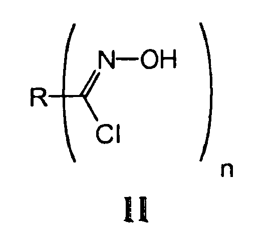 worin R und n die in Anspruch 1 angegebene Bedeutung haben,
mit einem Aziridin-Derivat der allgemeinen Formel III 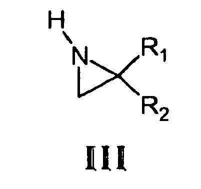 worin R₁ und R₂ die in Anspruch 1 angegebene Bedeutung haben, umsetzt

6. Arzneimittel, **gekennzeichnet durch** einen Gehalt an einer Verbindung gemäß einem der Ansprüche 1 bis 4.

7. Verwendung der 1-Aziridino-1-hydroxyiminomethyl-Derivate gemäß Anspruch 1 zur Herstellung von Arzneimitteln zur Behandlung von Tumoren oder von Krebserkrankungen.

8. Verwendung von 1,1'-[1,2-bis-(Hydroximino-)-1,2-ethandiyl]bis-aziridin zur Herstellung von Arzneimitteln zur Behandlung von Tumoren oder von Krebserkrankungen.

## Claims

1. 1-Aziridino-1-hydroxyiminomethyl derivatives of general formula I 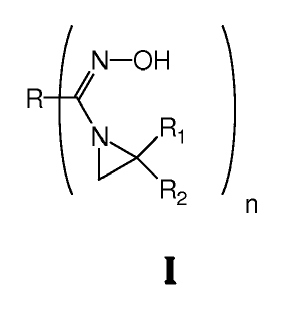 wherein
R is any organic moiety capable of bonding covalently two aziridine oxime groups and is selected from
a single bond, linear or branched saturated or unsaturated alkanes or heteroalkanes with up to 6 carbon atoms and with up to four hetero atoms, and C₃-C₈ cycloalkanes, that are optionally substituted with short-chain C₁-C₆ alkyl, C₁-C₆ alkoxy, nitro, amino, monosubstituted amino and/or halogen,
heterocyclic compounds with 3 to 6 ring atoms and up to four hetero atoms,
aromatic compounds with up to 8 ring atoms, optionally substituted with cyano, hydroxy, short-chain C₁-C₆ alkyl, C₁-C₆ alkoxy, nitro, amino, monosubstituted amino, trihaloalkyl and/or halogen and
heteroaryls with 3 to 7 ring atoms and up to four hetero atoms,
R₁ and R₂ independently of one another represent a hydrogen atom or a -CH₃, -C₂H₅, -CN, -COOH, -COOCH₃, -COOC₂H₅, -CONH₂ or C₆H₅ group,
n is the integer 2,
with the exception of 1,2-bis(aziridin-N-yl) glyoxim and 1,2-bis(2-methylaziridin-N-yl) glyoxime.

2. 1-Aziridino-1-hydroxyiminomethyl derivatives according to claim 1, **characterized in that** the main body R is selected from a single bond, methyl, ethane, ethene, ethine, propane, isopropane, butane, isobutane, sec-butane, pentane, isopentane, neopentane, hexane, azine, cylopropane, cyclobutane, cyclopentane, cyclohexane, cycloheptane, cyclooctane, pyrrole, pyrroline, pyrrolidine, imidazole, imidazoline, pyrazolidine, thiazole, thiazoline, thiazolidine, isothiazole, isothiazoline, isothiazolidine, benzothiazole, furan, dihyrofuran, tetrahydrofuran, benzofuran, thiophene, benzothiophene, oxazole, oxazoline, oxazolidine, benzoxazole, isoxazole, isoxazoline, isoxazolidine, piperidine, piperazine, pyrimidine, morpholine, dihydropyran, tetrahydropyran, pyridazine, benzene, furoxane, imidazolidine, pyrazole, pyrazoline, pyridine and the N-oxide thereof, dihydropyridine, pyrazine.

3. 1-Aziridino-1-hydroxyiminomethyl derivatives according to any of the preceding claims, **characterized in that** R₁ and R₂ independently of one another are hydrogen atoms or represent the moiety -CONH₂.

4. 1-Aziridino-1-hydroxyiminomethyl derivatives according to claim 1, namely
2,6-bis(1-aziridino-1-hydroxyiminomethyl)pyridine (6),
1,4-bis(1-aziridino-1-hydroxyiminomethyl)benzene (7),
1,4-di(α-2-carbamoylaziridino-α-hydroxyiminomethyl)-benzene (8),
1,3-bis(1-aziridino-1-hydroxyiminomethyl)benzene (9)
1,3-di(α-2-carbamoylaziridino-α-hydroxyiminomethyl)-benzene (11),
2,6-di(α-2-carbamoylaziridino-α-hydroxyiminomethyl)-pyridine (12),
3,5-bis(1-aziridino-1-hydroxyiminomethyl)pyridine (13),
2,5-bis(1-aziridino-1-hydroxyiminomethyl)pyridine (14),
2,4-bis(1-aziridino-1-hydroxyiminomethyl)pyridine (15),
2,5,-bis(1-aziridino-1-hydroxyiminomethyl)furan (16),
3,4-bis[(aziridinyl-1)-hydroxyiminomethyl]furoxane (17),
bis(2-methoxycarbonylaziridino)glyoxime (18),
bis(2-carbamoylaziridino)glyoxime (19),
2,2'-azinobis(1-aziridino-1-hydroxyimino)propane (20) and
2,2'-azinobis[1-(2-carbamoylaziridino)-1-hydroxy-imino]propane (21).

5. Method for preparing 1-aziridino-1-hydroxyiminomethyl derivatives according to claim 1, wherein in a manner known as such a halogen compound of general formula II 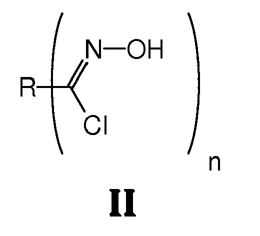 wherein R and n have the meaning cited in claim 1, is reacted with an aziridine derivative of general formula III 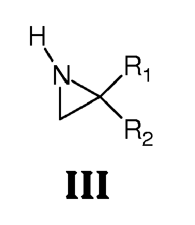 wherein R₁ and R₂ have the meaning cited in claim 1.

6. Pharmaceutical product, **characterized by** a content of a compound according to any of claims 1 to 4.

7. Use of the 1-Aziridino-1-hydroxyiminomethyl derivatives according to claim 1 for the preparation of pharmaceutical products for the treatment of tumours or cancerous diseases.

8. Use of 1,1'-[1,2-bis-(hydroximino-)-1,2-ethandiyl]bis-aziridin for the preparation of pharmaceutical products for the treatment of tumours or cancerous diseases.

## Revendications

1. Dérivés de 1-aziridino-1-hydroxyiminométhyle de formule générale I 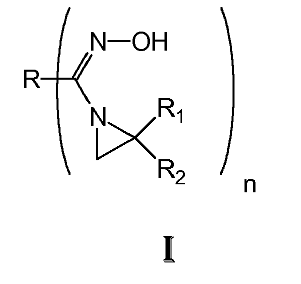 où
R signifie un radical organique quelconque, qui est en mesure de lier par covalence deux groupements aziridinoxime et qui est choisi parmi
- une simple liaison, des alcanes ou hétéroalcanes linéaires ou ramifiés et saturés ou insaturés, comprenant jusqu'à 6 atomes de carbone et jusqu'à quatre hétéroatomes, des C₃-C₈-cycloalcanes, qui sont le cas échéant substitués par des radicaux à courte chaîne C₁-C₆-alkyle, C₁-C₆-alcoxy, nitro, amino, amino monosubstitué et/ou halogène,
- des composés hétérocycliques comprenant 3 à 6 atomes de cycle et jusqu'à quatre hétéroatomes,
- des composés aromatiques comprenant jusqu'à 8 atomes de cycle qui sont le cas échéant substitués par des radicaux cyano, hydroxy, des radicaux à courte chaîne C₁-C₆-alkyle, C₁-C₆-alcoxy, nitro, amino, amino monosubstitué, trihalogénoalkyle et/ou halogène et
- des hétéroaryles comprenant 3 à 7 atomes de cycle et jusqu'à quatre hétéroatomes,
- R₁ et R₂ représentent, indépendamment l'un de l'autre, un atome d'hydrogène ou un groupe -CH₃, -C₂H₅, -CN, -COOH, -COOCH₃, -COOC₂H₅, -CONH₂ ou C₆H₅,
- n représente le nombre entier 2,
à l'exception de la 1,2-bis-(aziridin-N-yl)glyoxime et 1,2-bis-(2-méthylaziridin-N-yl)glyoxime.

2. Dérivés de 1-aziridino-1-hydroxyiminométhyle selon la revendication 1, **caractérisés en ce que** le corps de base R est choisi parmi une simple liaison, méthyle, éthane, éthène, éthyne, propane, isopropane, butane, isobutane, sec-butane, pentane, isopentane, néopentane, hexane, azine, cyclopropane, cyclobutane, cyclopentane, cyclohexane, cycloheptane, cyclooctane, pyrrole, pyrroline, pyrrolidine, imidazole, imidazoline, pyrazolidine, thiazole, thiazoline, thiazolidine, isothiazole, isothiazoline, isothiazolidine, benzothiazole, furanne, dihydrofuranne, tétrahydrofuranne, benzofuranne, thiophène, benzothiophène, oxazole, oxazoline, oxazolidine, benzoxazole, isoxazole, isoxazoline, isoxazolidine, pipéridine, pipérazine, pyrimidine, morpholine, dihydropyranne, tétrahydropyranne, pyridazine, benzène, furoxanne, imidazolidine, pyrazole, pyrazoline, pyridine et son N-oxyde, dihydropyridine, pyrazine.

3. Dérivés de 1-aziridino-1-hydroxyiminométhyle selon l'une quelconque des revendications précédentes, **caractérisés en ce que** R₁ et R₂ représentent, indépendamment l'un de l'autre, des atomes d'hydrogène ou un radical -CONH₂.

4. Dérivés de 1-aziridino-1-hydroxyiminométhyle selon la revendication 1, notamment
2,6-bis-(1-aziridino-1-hydroxyiminométhyl)pyridine (6),
1,4-bis-(1-aziridino-1-hydroxyiminométhyl)benzène (7),
1,4-di-(α-2-carbamoylaziridino-α-hydroxyiminométhyl)- benzène (8),
1,3-bis-(1-aziridino-1-hydroxyiminométhyl)benzène (9),
1,3-di-(α-2-carbamoylaziridino-α-hydroxyiminométhyl)-benzène (11),
2,6-di-(α-2-carbamoylaziridino-α-hydroxyiminométhyl)-pyridine (12),
3,5-bis-(1-aziridino-1-hydroxyiminométhyl)pyridine (13),
2,5-bis-(1-aziridino-1-hydroxyiminométhyl)pyridine (14),
2,4-bis-(1-aziridino-1-hydroxyiminométhyl)pyridine (15),
2,5-bis-(1-aziridino-1-hydroxyiminométhyl)furanne (16),
3,4-bis-[(aziridinyl-1)-hydroxyiminométhyl]furoxanne (17),
bis-(2-méthoxycarbonylaziridino)glyoxime (18),
bis-(2-carbamoylaziridino)glyoxime (19),
2,2'-azinobis(1-aziridino-1-hydroxyimino)propane (20) et
2,2'-azinobis[1-(2-carbamoylaziridino)-1-hydroxyimino]propane (21).

5. Procédé pour la préparation de dérivés de 1-aziridino-1-hydroxyiminométhyle selon la revendication 1, où on transforme de manière connue en soi un composé halogéné de formule générale II 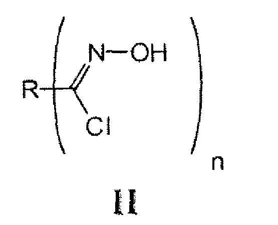 dans laquelle R et n ont la signification indiquée dans la revendication 1, avec un dérivé d'aziridine de formule générale III 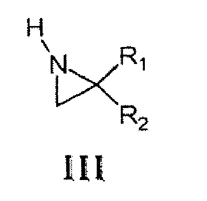 dans laquelle R₁ et R₂ ont la signification indiquée dans la revendication 1.

6. Médicament, **caractérisé par** une teneur en un composé selon l'une quelconque des revendications 1 à 4.

7. Utilisation des dérivés de 1-aziridino-1-hydroxyiminométhyle selon la revendication 1 pour la préparation de médicaments destinés au traitement de tumeurs ou de maladies cancéreuses.

8. Utilisation de 1,1'-[1,2-bis-(hydroximino-)-1,2-éthanediyl]bisaziridine pour la préparation de médicaments destinés au traitement de tumeurs ou de maladies cancéreuses.
